Europäisches Patentamt

**European Patent Office**

**Office européen des brevets**

(11) Publication number: **0 201 776**

**A2**

## (12) EUROPEAN PATENT APPLICATION

(21) Application number: **86105692.7**

(22) Date of filing: **24.04.86**

(51) Int. Cl.4: **G01N 27/56** , G01N 33/18

(30) Priority: **14.05.85 IT 6744085**

(43) Date of publication of application:
**20.11.86 Bulletin 86/47**

(84) Designated Contracting States:
**AT BE CH DE FR GB LI LU NL SE**

(71) Applicant: **SIGNORETTO STAMPI S.p.A.**
**Corso Vinzaglio, 33**
**I-10121 Torino(IT)**

(72) Inventor: **Michelis, Bruno**
**Viale Piaggio, 23**
**I-12078 Ormea(IT)**

(74) Representative: **Prato, Roberto et al**
**c/o Ingg. Carlo e Mario Torta Via Viotti 9**
**I-10121 Torino(IT)**

(54) **Process and apparatus for automatically measuring the acidity of atmospheric precipitation.**

(57) Precipitation is collected in a damp-electrode electrolytic cell (3) and the pH of the precipitation measured using a measuring instrument (22); precipitation is fed into the cell (3) from a collecting vessel (7) with a movable normally-closed lid (15) which is opened as soon as a water sensor (11) on another permanently-open precipitation collecting vessel (6) transmits a signal to a programmable controller (40) controlling the lid (15); when precipitation ceases, the controller (40) closes the lid (15) and, by means of a pump (34), feeds a rinsing and moisturizing solution from a container (31) into the cell (3), whereas the precipitation collected in the cell (3) is drained off by means of a valve (28).

# PROCESS AND APPARATUS FOR AUTOMATICALLY MEASURING THE ACIDITY OF ATMOSPHERIC PRECIPITATION

The present invention relates to a process for automatically measuring the acidity (pH) of atmospheric precipitation, particularly rainwater in urban and/or polluted areas, as well as an apparatus for conducting the said process.

At present, ecological control of atmospheric precipitation is known to be conducted manually, by collecting samples which are then laboratory tested for determining the pH value. In addition to being expensive, such control is also inefficient in that the samples collected are not always significant.

The aim of the present invention is to provide a process for automatically sampling and measuring the pH of atmospheric precipitation, in such a manner as to obtain significant results.

A further aim of the present invention is to provide an apparatus for conducting the said process.

With these aims in view, according to the present invention, there is provided a process for automatically measuring the acidity of atmospheric precipitation, characterised by the fact that it comprises stages consisting in:

-continuously detecting the presence of water in a first precipitation collecting vessel, the access opening of which is permanently open, and which is provided with a free drain;

-activating, subsequent to the detection of water in the said first collecting vessel, a damp-electrode pH-measuring electrolytic cell, by emptying the said cell of the moisturising solution contained inside it;

-opening a cover over an access opening on a second normally-closed precipitation collecting vessel connected hydraulically to the said pH-measuring cell;

-measuring the pH of the precipitation collected inside the said measuring cell, once the liquid inside the said cell reaches a given preset level;

-closing the said cover and filling the said measuring cell with the said moisturising solution, after first emptying the said cell of the precipitation collected inside it, subsequent to no more water being detected in the said first collecting vessel.

The present invention also relates to an apparatus for automatically measuring the acidity of atmosphere precipitation, characterised by the fact that it comprises in due combination : a pair of vessels for collecting the said precipitation, a first of which vessels is permanently open to the atmosphere and provided internally with a water sensor, and a second of which vessels is provided with an access opening closed by means of a movable lid opened and closed by means of a servomotor; a damp-electrode pH-measuring electrolytic cell connected hydraulically to the said second precipitation collecting vessel and having a measuring instrument, a drain opening controlled by a valve, and a level sensor; at least one container containing a moisturising solution for the said cell and provided with a pump and a pipe for feeding the said solution to the said electrolytic cell; and a control system for selectively activating the said servomotor, valve and pump, according to the signals supplied by the said level sensor and the said water sensor.

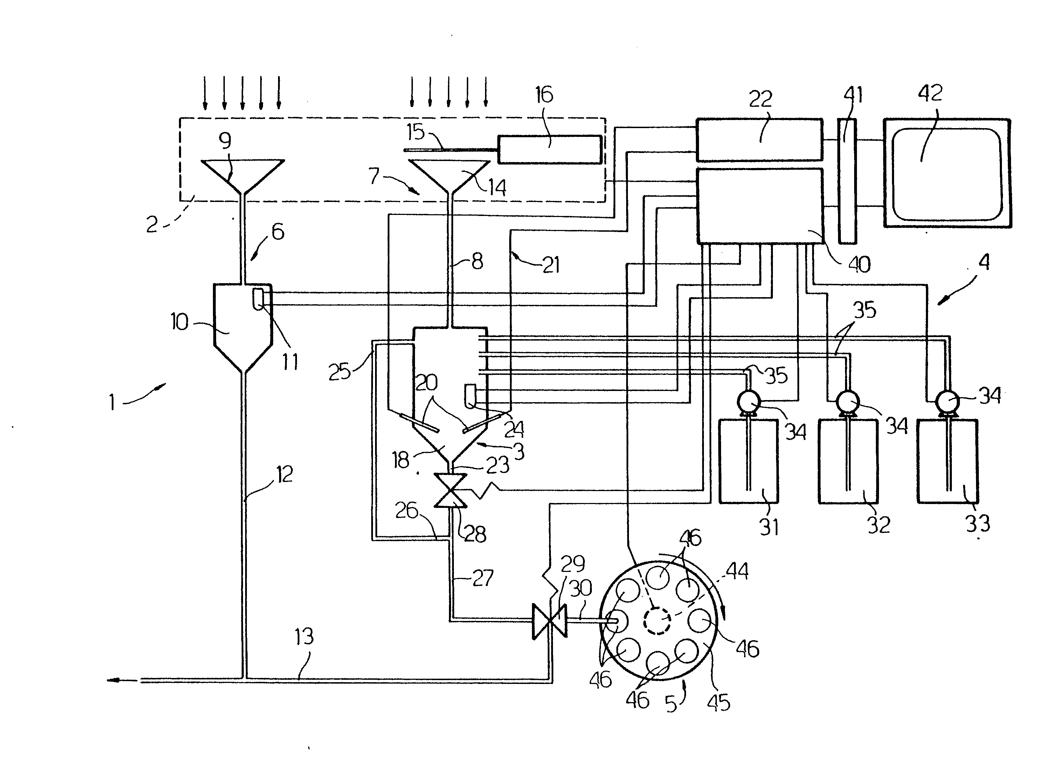

The present invention will be described by way of example with reference to the attached drawing showing a schematic view of the apparatus according to the present invention. Number 1 in the aforementioned drawing indicates an apparatus for automatically detecting the acidity (pH) of precipitation caused by atmospheric phenomena, such as rain showers, storms and similar. Apparatus 1 comprises a collecting unit 2 shown schematically by the dotted line in the attached drawing; an electrolytic pH-measuring cell 3 of any known type, particularly the damp-electrode type normally employed in laboratories; control and service devices 4; and an optical sampling device 5. Collecting unit 2 comprises a first vessel 6 for collecting precipitation (shown schematically by the arrows in the attached drawing), and a second collecting vessel 7 alongside vessel 6 and connected hydraulically, by means of pipe 8, to measuring cell 3, which is preferably located below vessel 7 to enable any liquid inside vessel 7 to drip by force of gravity into cell 3. Vessel 6 is permanently open to the atmosphere, no covering being provided on its funnel-shaped top access opening 9, and is provided internally, e.g. in an accumulating chamber 10, with a water sensor 11 of any known type and preferably capacitive. Collecting vessel 6 is also provided with a gravity drain pipe 12 by means of which the precipitation collected in chamber 10 is drained off continuously into a free drain pipe 13 into which pipe 12 comes out. Vessel 7, on the other hand, is normally closed in that, according to the present invention, it is provided with a funnel-shaped top access opening 14 closed by a movable, e.g. sliding, lid 15 opened and closed in known manner by a preferably electric servomotor 16.

Cell 3 comprises a bulb 18, made of nonconducting material and containing respective reference and measuring electrodes 20 of known type and therefore not described in detail for the sake of simplicity, and a known type of electricity supply and measuring circuit 21 comprising a measuring instrument 22 preferably consisting of an electronic pH-meter. Bulb 18, into which pipe 8 comes out, presents a bottom drain opening 23, a known type of level sensor 24, and an overflow 25, one drain pipe 26 of which comes out inside a pipe 27 downstream from an open/close valve 28 controlling drain opening 23 and preferably consisting of a solenoid gate valve. Along pipe 27, provision is made for a switch valve 29, preferably consisting of a three-way solenoid valve, for selectively connecting drain opening 23, and in particular pipe 27 with which opening 23 communicates via valve 28, with pipe 13 or with a second free drain pipe 30 for supplying sampling device 5.

Control and service devices 4 comprise three respective containers 31, 32 and 33 for respectively storing a moisturising solution for cell 3 and two different buffer solutions for setting pH-meter 22, said buffer solutions having different known pH values and being selected according to the pH values being measured. For example, in the case of a cell 3 with a calomel reference electrode 20, the moisturising solution will consist of distilled water, and, assuming pH measurements in the 2 to 7 range, typical of rainwater in polluted environments, the buffer solutions in containers 32 and 33 will be selected so as to present a constant pH of 4 and 7 respectively. In any case, however, the moisturising and buffer solutions stored in containers 31, 32 and 33 are of known types, being identical to those normally employed in laboratores for the same purposes, i.e. for maintaining the reference electrode moist between successive pH measurements, and for taking pH measurements of known-pH samples, for determining any deviation in the measurement and for setting instrument 22.

Each container 31, 32 and 33 is provided with a respective electropump 34 for feeding the respective solution inside the container into cell 3 via respective supply pipes 35 which come out inside bulb 18. According to the present invention, pumps 34, valves 23 and 29 and servomotor 16 are controlled by a control system 40 preferably consisting of a commercial type programmable controller or a specially programmed and equipped microprocessor, which sys tem is also designed to receive and process signals supplied by sensors 11 and 24, and is connected to instrument 22 via a known type of input/output device 41, which preferably connects both system 40 and instrument 22 to a local or remote recording set 42 and/or to a known type of manually-operated remote control unit shown for the sake of simplicity. System 40 also controls a servomotor 44 on optional sampling device 5 (if provided). Device 5 comprises an indexing fixture 45 powered by servomotor 44, and a number of containers 46 of given volume, mounted integral with fixture 45 which is designed to set containers 46 selectively next to the outlet of pipe 30 for collecting any drainage from the same. If necessary, containers 46 may also be provided with level sensors of the same type as sensor 24 and connected to system 40 in such a manner as to cause fixture 45 to turn when the container next to the outlet of pipe 30 is full. Also, cell 3 may be provided with electrical temperature-compensating resistors.

According to the present invention, system 40 is programmed so as to perform the sequence of operations hereinafter described and which constitutes the stages in the measuring process covered by the present invention. Via sensor 11, system 40 checks continuously whether or not water is being collected inside chamber 10. The absence of water inside chamber 10 indicates the absence of precipitation, in which case, system 40 keeps lid 15 closed and bulb 18 full of moisturising solution, so as to safeguard the efficiency of the reference electrode on cell 3 and prevent contaminating substances from depositing inside vessel 7. In the event of an atmospheric phenomenon producing precipitation, said precipitation is partly collected in vessel 6, and water is collected inside chamber 10 and detected by sensor 11. Subsequent to the detection of water in chamber 10, system 40 activates motor 16 for opening lid 15 and, at the same time, activates cell 3 for measuring by opening valve 28 and so draining the distilled water - (moisturising solution) from bulb 18 through opening 23. During this stage, valve 29 is regulated by system 40 so as to drain pipe 27 into pipe 13 and so cut off device 5.

Subsequent to the opening of lid 15, atmospheric precipitation is collected in vessel 7 from which it drips down into bulb 18, the level of the said precipitation gradually rising until overflow 25 is reached. When the said preset level is reached (preferably corresponding to a collected precipitation volume of 20 cc) it is signalled by sensor 24 to system 40 which switches valve 29 (valve 28 having obviously already been closed subsequent to sensor 24 signalling complete emptying of bulb 18) and activates instrument 22 for measuring the pH value which is recorded on recorder 42. During this stage, precipitation in excess of the volume of bulb 18 is drained into pipe 30 and collected inside one of containers 46 on sampling device 5 (if provided), or simply drained off if sampling device 5 is not provided for. Once the measuring stage has been completed, system 40 opens valve 28 so as to also drain off, via pipe 30, the liquid collected inside bulb 18 and the pH of which has been measured, after which, it closes valve 28 and prepares for another measurement as soon as bulb 18 fills up again. If the amount of precipitation involved is greater than a given preset threshold, this is detected by sensor 11 and system 40 may switch cell 3 to continuous measuring mode. In this case, valve 28 stays closed and recorder 42 records the instantaneous real-time pH value of the precipitation, thus enabling development of the same to be followed more closely. Finally, when precipitation ceases, vessel 6, which is drained freely, is emptied and sensor 11 signals the absence of water in chamber 10. When the said signal from sensor 11 is received, system 40 cuts off the measuring function, closes lid 15, empties cell 3, turns fixture 45 (if provided) so that only the precipitation relative to a given atmospheric phenomenon is collected inside each container 46, switches valve 29 so as to drain pipe 27 into pipe 13, and starts up pump 34 of container 31 so as to rinse cell 3 with moisturising solution and, after first closing valve 28, fill it with the same. At this point, apparatus 1 switches to "standby" mode awaiting further precipitation, during which time, at preset intervals set on system 40 or as requested by the operator - (using the said remote controls) it sets instrument 22 by emptying cell 3 of the moisturising solution and, via pumps 34, filling it with the two different-pH buffer solutions in containers 32 and 33. Once cell 3 has been filled and such indicated by sensor 24, system 40 measures the pH of each buffer solution and compares the result with the values set and memorised in system 40 by the operator, instrument 22 then being set by system 40 according to the difference detected.

In the event of further precipitation commencing during this stage, such is indicated by sensor 11, and system 40, after abandoning the setting function and rinsing cell 3, commences another measuring cycle.

The advantages of the present invention will be clear from the foregoing description. To those skilled in the art it will also be clear that changes may be made to the apparatus described herein without, however, departing from the scope of the present invention.

## Claims

1) -A process for automatically measuring the acidity of atmospheric precipitation, characterised by the fact that it comprises stages consisting in :

-continuously detecting the presence of water in a first precipitation collecting vessel (6), the access opening (9) of which is permanently open, and which is provided with a free drain (12);

-activating, subsequent to the detection of water in the said first collecting vessel (6), a damp-electrode pH-measuring electrolytic cell (3), by emptying the said cell (3) of the moisturising solution contained inside it;

-opening a cover (15) over an access opening (14) on a second normally-closed precipitation collecting vessel (7) connected hydraulically to the said pH-measuring cell (3);

-measuring the pH of the precipitation collected inside the said measuring cell (3), once the liquid inside the said cell (3) reaches a given preset level;

-closing the said cover (15) and filling the said measuring cell (3) with the said moisturising solution, after first emptying the said cell (3) of the precipitation collected inside it, subsequent to no more water being detected in the said first collecting vessel (6).

2) -A process as claimed in Claim 1, characterised by the fact that the pH measurements are recorded on a recorder (42) and that the atmospheric precipitation drained from the said measuring cell (3) is collected in a sampling device (5) having a number of containers (46) of given volume.

3) -A process as claimed in Claim 1 or 2, characterised by the fact that it also comprises a stage for setting a measuring instrument (22) forming part of the said measuring cell (3), in which stage, two different buffer solutions having different known pH values are fed into the said measuring cell (3), after first rinsing the said measuring cell (3) with the said moisturising solution, for the purpose of measuring its pH value, the measured pH values subsequently being compared with known values for setting the said measuring instrument (22).

4) -A process as claimed in one of the foregoing Claims from 1 to 3, characterised by the fact that the said stages are performed by activating servomechanisms (16, 44), respective pumps (34) and electrovalves (28, 29) by means of a programmable controller (40) connected to appropriate sensors - (11, 24).

5) -An apparatus (1) for automatically measuring the acidity of atmospheric precipitation, characterised by the fact that it comprises in due combination : a pair of vessels (6, 7) for collecting the said precipitation, a first of which vessels is permanently open to the atmosphere and provided internally with a water sensor (11), and a second of which vessels is provided with an access opening - (14) closed by means of a movable lid (15) opened and closed by means of a servomotor (16); a damp-electrode pH-measuring electrolytic cell (3) connected hydraulically to the said second precipitation collecting vessel (7) and having a measuring instrument (22), a drain opening (23) controlled by a valve (28), and a level sensor (24); at least one container (31) containing a moisturising solution for the said cell (3) and provided with a pump (34) and a pipe (35) for feeding the said solution to the said electrolytic cell (3); and a control system (40) for selectively activating the said servomotor (16), valve (28) and pump (34), according to the signals supplied by the said level sensor (24) and the said water sensor (11).

6) -An apparatus (1) as claimed in Claim 5, characterised by the fact that it also comprises a switch valve (29) located downstream from the said valve (28) controlling the drain opening (23) on the said measuring cell (3), said valve (29) being designed for selectively connecting the said drain opening - (23) to a first or second drain pipe (13, 30), the said first pipe (13) being connected to a drain pipe (12) on the said first precipitation collecting vessel - (6), and the drain pipe of an overflow (25) on the said measuring cell (3) being connected upstream from the said switch valve (29).

7) - An apparatus (1) as claimed in Claim 6, characterised by the fact that it also comprises a sampling device (5) at the outlet of the said second drain pipe (30), the said sampling device (5) comprising an indexing fixture (45) driven by a second servomotor (44) controlled by the said system (40), and a number of containers (46) of given volume mounted on the said indexing fixture (45), the said indexing fixture (45) being designed to bring the said containers (46) selectively up to the said outlet of the said second drain pipe (30) for collecting in the said containers (46) the said atmospheric precipitation.

8) -An apparatus (1) as claimed in one of the foregoing Claims from 5 to 7, characterised by the fact that it comprises a further two containers (32, 33) containing two different buffer solutions for setting the said measuring instrument (22), the said containers (32, 33) being provided with respective pumps (34) for feeding the said buffer solutions into the said measuring cell (3) via respective supply pipes (35), and the said pumps (34) being controlled by the said system (40) in response to signals from the said level sensor (24).

9) -An apparatus (1) as claimed in one of the foregoing Claims from 4 to 7, characterised by the fact that it is designed to perform the process as claimed in Claims 1 to 4.